(19) Europäisches Patentamt
European Patent Office
Office européen des brevets

(11) EP 0 683 216 B1

(12) EUROPEAN PATENT SPECIFICATION

(45) Date of publication and mention of the grant of the patent:
**23.08.2000 Bulletin 2000/34**

(51) Int Cl.[7]: **C09J 7/02**, C09J 7/00, A61L 15/58, A61F 7/00, A61F 13/15

(21) Application number: **95107274.3**

(22) Date of filing: **12.05.1995**

(54) **Re-peelable pressure-sensitive adhesive, pressure-sensitive adhesive tape or sheet, and fastening system employing these**

Wiederabziehbar Haftklebe, Haftklebeband oder Haftklebefilm, und Befestigungsystem mit diesem System

Adhésif repelable et sensible à la pression, bande ou film adhésif sensible à la pression et système de fixation les utilisant

(84) Designated Contracting States:
**BE DE FR GB**

(30) Priority: **17.05.1994 JP 10291294**
**24.06.1994 JP 14317894**
**29.03.1995 JP 7118295**

(43) Date of publication of application:
**22.11.1995 Bulletin 1995/47**

(73) Proprietor: **NITTO DENKO CORPORATION**
**Osaka (JP)**

(72) Inventors:
- **Arakawa, Masaaki**
  **Ibaraki-shi, Osaka (JP)**
- **Morimoto, Yuuchi**
  **Ibaraki-shi, Osaka (JP)**

(74) Representative: **Grünecker, Kinkeldey, Stockmair & Schwanhäusser Anwaltssozietät**
**Maximilianstrasse 58**
**80538 München (DE)**

(56) References cited:
**EP-A- 0 306 232**

- **PATENT ABSTRACTS OF JAPAN vol. 18 no. 40 (C-1155) [6380] ,21 January 1994 & JP-A-05 263056 (ASASHI CHEM IND CO LTD)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 683 216 B1

## Description

### FIELD OF THE INVENTION

The present invention relates to a re-peelable pressure-sensitive adhesive according to the preamble of claim 1. The present invention also relates to a fastening system employing these. Although applications thereof are not particularly limited, they are suitable for use, e.g., as a fastening system for disposable diapers, for fixing sanitary napkins or sealing packages thereof, for opening or closing plastic bags or packages (boxes, bags, etc.), for fixing disposable pocket heaters of the adhesive type, for fixing seals, emblems, or labels to garments, fabrics, nonwoven fabrics, etc., or in pressure-sensitive adhesive tape rolls in which the base material is not coated on its back side with a releasing agent.

### BACKGROUND OF THE INVENTION

In the field where pressure-sensitive adhesive tapes or the like are used to fix, re-peel, and re-fix various adherends, the adhesive force of the conventional pressure-sensitive adhesive tapes to the adherends (releasing force) increases from the initial adhesive force due to the lapse of time for storage, a temperature increase after pressure-sensitive adhesive application, or other factors. When it is attempted to peel off the applied pressure-sensitive adhesive tapes after the lapse of a certain time period from application, as in the case of removal after use or changing the application position, the increased adhesive force causes problems that peeling is difficult, the adherend is damaged, the base material of the pressure-sensitive adhesive tape or the like breaks, and the pressure-sensitive adhesive is left to foul the adherend.

Furthermore, since conventional pressure-sensitive adhesive tapes and the like are usually designed so that the releasing forces thereof become higher with increasing peel rate, most of these have a problem that the adherends break especially when the adherends have low strength (e.g., the back sheets of disposable diapers).

For example, various pressure-sensitive adhesive compositions for fastening and unfastening disposable paper diapers are known. For example, a pressure-sensitive adhesive composition comprising an A-B-A block copolymer such as a styrene-isoprene-styrene block copolymer, a solid tackifier resin, a liquid tackifier resin, and a terminal-block reinforcing resin has been proposed (JP-A-1-95175); this composition is intended to have the maximum releasing force at a specific peel rate. (The term "JP-A" as used herein means an "unexamined published Japanese patent application.") However, use of this pressure-sensitive adhesive composition for, e.g., fastening and unfastening a disposable paper diaper poses a problem that after application of the pressure-sensitive adhesive tape, the adhesive force to the adherend increases considerably from the initial adhesive force as a result of the lapse of time for storage or due to a temperature increase, etc., as described above. As a result, peeling of the pressure-sensitive adhesive tape becomes difficult especially because the releasing force is maximum at a high peel rate. Moreover, if the pressure-sensitive adhesive tape is applied directly to the back sheet of a paper diaper, the back sheet, which is made of polyethylene or the like, tends to break. In addition, the conventional pressure-sensitive adhesive tape is still insufficient in overcoming problems concerning the diminution of peeling noise, prevention of adhesive residue, etc.

On the other hand, adhesive-type disposable pocket heaters employing a pressure-sensitive adhesive are known. However, these conventional pocket heaters have a problem that the adhesive force (releasing force) of the pressure-sensitive adhesive increases with the lapse of time after application to a garment or another adherend because of, e.g., a temperature increase. As a result, peeling off the pocket heater after the lapse of a certain time period as in the case of removal after use leaves the pressure-sensitive adhesive on the garment, damages the garment, etc., or causes another trouble.

### SUMMARY OF THE INVENTION

The present invention has been achieved in order to mitigate the conventional problems. An object of the present invention is to provide a re-peelable pressure-sensitive adhesive, a pressure-sensitive adhesive tape or sheet, or a fastening system employing these with each of which sufficient bonding and fixing and easy re-peeling after use or for position changing can be attained by use of a pressure-sensitive adhesive which after application undergoes less increase in adhesive force with the lapse of time, a temperature change, etc., i.e., a pressure-sensitive adhesive which has less difference between the initial adhesive force (releasing force) just after application and the adhesive force (releasing force) at the time of re-peeling, removal after use, etc.

The above problem is solved according to the present invention by a re-peelable pressure-sensitive adhesive which comprises the features set out in claim 1.

BRIEF DESCRIPTION OF THE DRAWINGS

Fig. 1 is a sectional view showing one embodiment of the pressure-sensitive adhesive tape of the present invention.

Fig. 2 is a sectional view showing another embodiment of the pressure-sensitive adhesive tape of the present invention.

Fig. 3 is a sectional view showing still another embodiment of the pressure-sensitive adhesive tape of the present invention.

Fig. 4 is a sectional view showing a further embodiment of the pressure-sensitive adhesive tape of the present invention.

Fig. 5 is a sectional view showing still a further embodiment of the pressure-sensitive adhesive tape of the present invention.

Fig. 6 is a schematic view showing one embodiment of a disposable diaper having the pressure-sensitive adhesive tape of the present invention.

Fig. 7 is a schematic view showing one embodiment of a sanitary napkin package having the pressure-sensitive adhesive tape of the present invention.

Fig. 8 is a schematic view showing one embodiment of a separator-less sanitary napkin package having the pressure-sensitive adhesive tape of the present invention.

Fig. 9 is a schematic view showing one embodiment of a disposable pocket heater employing the pressure-sensitive adhesive sheet of the present invention.

Fig. 10 is a schematic view showing one embodiment of a package having the pressure-sensitive adhesive tape of the present invention.

Fig. 11 is a schematic view showing one embodiment of a plastic bag having the pressure-sensitive adhesive tape of the present invention.

Fig. 12 is a schematic view showing one embodiment of a pressure-sensitive adhesive tape roll produced through coating with the re-peelable pressure-sensitive adhesive of the present invention without using a releasing agent.

Fig. 13 is a graph showing an example of the relationship between peel rate and releasing force (adhesive force) in the present invention.

DETAILED DESCRIPTION OF THE INVENTION

It is important that the re-peelable pressure-sensitive adhesive in the present invention and the pressure-sensitive adhesive tape or sheet (hereinafter often referred to as "pressure-sensitive adhesive sheet or the like") obtained by forming a layer of the re-peelable pressure-sensitive adhesive on any desired base material be designed so that the adhesive force of the pressure-sensitive adhesive or pressure-sensitive adhesive tape or the like to an adherend at the time of re-peeling (releasing force) is up to 4 times, desirably up to 3 times, preferably about from 1.2 to 2.0 times, the initial adhesive force as measured in the initial stage after application. The pressure-sensitive adhesive is not particularly limited in composition, etc., as long as it has this performance. Adhesive forces elevated to above 4 times the initial value are undesirable in that the adhesive force (releasing force) is so high that the adherend such as, e.g., a garment, a diaper, or a napkin may be damaged or the base material of the tape or sheet may break. The initial adhesive force herein means the adhesive force at the time of peeling within about 30 minutes after application.

In this invention, the pressure-sensitive adhesive sheet or the like or the pressure-sensitive adhesive desirably has such a property that when it is applied to an adherend such as, e.g., a garment, a diaper, or a napkin and peeled off thereafter, its adhesive force (releasing force) to the adherend (mostly cotton fabric) decreases with increasing peel rate when the peel rate is not lower than 3 m/min, preferably about from 5 to 10 m/min. The property of undergoing a decrease in adhesive force (releasing force) in that peel rate range brings about the effect of attaining easier peeling in practical use, because the rates of manual peeling are around 10 m/min for most persons and are 20 to 30 m/min for quick-peeling persons.

It is also preferred in the present invention that the releasing force (adhesive force) at a high peel rate of 10 m/min or higher, particularly about from 10 to 100 m/min, be equal to or lower than the releasing force at a low peel rate of 0.1 m/min or lower. The reason for this is as follows. In the case where the re-peelable pressure-sensitive adhesive or the like of the present invention is used, for example, in the fastening system of a disposable diaper which will be described later, the diaper is sufficiently fastened and is less apt to be unnecessarily unfastened at the time of the initiation of peeling by, e.g., the mother or during wearing (low-peel-rate range) since the pressure-sensitive adhesive has a high releasing force in this low-peel-rate range, while the pressure-sensitive adhesive can be extremely easily peeled off without breaking the diaper's back sheet as an adherend since it has a reduced releasing force during peeling (high-peel-rate range). Thus, a fastening system preferable for practical use is obtained.

In particular, in the case where the pressure-sensitive adhesive tape of the present invention is directly applied to the back sheet of a diaper, the preferred range of the adhesive force of the adhesive tape depends on the strength of the back sheet. If the strength of the back sheet is 600 g/25-mm (breaking strength in elongation at 0.3 m/min), the adhesive force of the adhesive tape is desirably not higher than the back sheet strength over the range of from a low peel rate (0.1 m/min) to a high peel rate (100 m/min) from the standpoint of avoiding sheet breakage. The pressure-sensitive adhesive tape is also required not to undergo a decrease in adhesion with increasing number of re-peeling operations. For example, the percentage decrease for up to the third peeling operation based on the adhesive force at the first peeling operation is desirably 40% or lower. Too high percentage decreases in adhesive force may result in unnecessary unfastening.

More specifically, the pressure-sensitive adhesive tape is desirably designed so that the adhesive force thereof to, e.g., a cotton fabric as an underwear in the initial stage (just after application; e.g., ordinary temperature (23°C) or lower) is usually about from 400 to 800 g/25-mm and the adhesive force thereof to the adherend at the time of peeling (e.g., after the lapse of time, or after a temperature increase (to 50-60°C) and then a decrease to ordinary temperature) is about from 500 to 1,200 g/25-mm (at a peel rate of 0.3 m/min). Although such properties are desirable from the standpoints of reliable fastening, influence on the adherend, easiness of peeling, etc., the adhesion properties of the pressure-sensitive adhesive tape are not limited thereto.

The composition of the re-peelable pressure-sensitive adhesive in the present invention is not particularly limited as long as the pressure-sensitive adhesive satisfies the property requirements described above. It is, however, desirable in the present invention that the pressure-sensitive adhesive should contain (1) an elastomer which is a block copolymer at least comprising a polymer block A consisting mainly of units derived from a vinylaromatic compound and a polymer block B consisting mainly of units derived from a conjugated diene compound and (2) a tackifier resin.

It is preferred that the elastomer has a weight-average molecular weight of 50,000 to 300,000, more preferably from 100,000 to 200,000. Preferred examples of the elastomer include synthetic styrene block copolymers such as styrene-isoprene-styrene block copolymers (SIS), styrene-butadiene-styrene block copolymers (SBS), and hydrogenated copolymers obtained therefrom (SIPS, SEBS). These block copolymers may be used alone or as a mixture of two or more thereof.

In the present invention, the elastomer described above is especially preferably a block copolymer which comprises a polymer block A consisting mainly of units derived from a vinylaromatic compound and a polymer block B consisting mainly of units derived from a conjugated diene compound, and which has a block A content of 18% by weight or higher, or/and has a degree of coupling of 90% or higher, or/and is a radial block copolymer having three or more terminal polymer blocks A.

The content of the polymer block A (e.g. the content of styrene) in the block copolymer is preferably 18% by weight or higher, especially preferably about from 20 to 30% by weight. The reason for the preference of higher polymer block A contents is as follows. Higher polymer block A contents heighten the modulus of elasticity of the pressure-sensitive adhesive so that the penetration of the pressure-sensitive adhesive into an adherend, e.g., fibers, (enhanced wettability) can be prevented. As a result, the adhesive force of the pressure-sensitive adhesive can be prevented from increasing and the pressure-sensitive adhesive can be lightly peeled off when desired.

Examples of the above-described elastomer include the block copolymers available under the trade names of Quintac SH-108 (manufactured by Nippon Zeon Co., Ltd., Japan; styrene-isoprene-styrene block copolymer; styrene content, 25 wt%), Quintac 3450 (manufactured by Nippon Zeon Co., Ltd.,; styrene-isoprene-styrene block copolymer; styrene content, 19 wt%), Vector V-4111D (manufactured by Tornex Co.; styrene-isoprene-styrene block copolymer; styrene content, 18 wt%), Vector V-4211D (manufactured by Tornex Co.; styrene-isoprene-styrene block copolymer; styrene content, 30 wt%), RP-6405 (manufactured by Shell Kagaku K.K., Japan; styrene-isoprene-styrene block copolymer; styrene content, 30 wt%), and RP-6404 (manufactured by Shell Kagaku K.K.; styrene-isoprene-styrene block copolymer; styrene content, 30 wt%). However, the elastomer should not be construed as being limited to these examples.

Another preferred elastomer is one consisting mainly of a block copolymer which comprises a polymer block A consisting mainly of units derived from a vinylaromatic compound and a polymer block B consisting mainly of units derived from a conjugated diene compound, e.g., a styrene block copolymer, and which has a degree of coupling (the proportion by weight of triblocks in all blocks) of 90% or higher, preferably 93% or higher. The degree of coupling of the copolymer can be obtained according to the equation:(the amount by weight of triblock ABA in the copolymer / the total amount by weight of triblock ABA and diblock AB in the copolymer) $\times$ 100 (%). The triblock ABA is prepared by coupling diblocks AB composed of polymer blocks A and B to constitute the copolymer.

The triblocks in this copolymer constitute a domain structure (blocks of the vinylaromatic compound) to thereby serve like crosslinking. Hence, block copolymers having a small triblock amount (a low degree of coupling) have a drawback that they have a reduced cohesive force and a reduced modulus of elasticity, so that wettability to the adherend is enhanced, resulting in an increase in adhesive force. It is therefore thought that by using the copolymer described above, stringiness which occurs due to softening of pressure-sensitive adhesives as a result of the lapse of

time or upon heating etc. is reduced and the pressure-sensitive adhesive can be peeled off lightly.

Examples of the above-described elastomer include the block copolymers available under the trade names of Vector V-4111D (manufactured by Tornex Co.; styrene-isoprene-styrene block copolymer; degree of coupling, 99% or higher), Vector V-4211D (manufactured by Tornex Co.; styrene-isoprene-styrene block copolymer; degree of coupling, 99% or higher), Kraton D-1114X (manufactured by Shell Kagaku K.K.; styrene-isoprene-styrene block copolymer; degree of coupling, 100%), and Kraton D-1320X (manufactured by Shell Kagaku K.K.; styrene-isoprene-styrene block copolymer; degree of coupling, 90%). However, the elastomer should not be construed as being limited to these examples.

Still another preferred elastomer contained in the pressure-sensitive adhesive of the present invention is a block copolymer which comprises a polymer block A consisting mainly of units derived from a vinylaromatic compound and a polymer block B consisting mainly of units derived from a conjugated diene compound and which is a radial block copolymer having three or more terminal polymer blocks A.

The radial block copolymers having three terminal polymer blocks A and four terminal polymer blocks A can be illustrated by

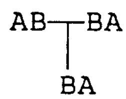

and

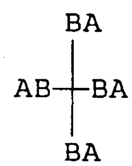

,

respectively.

In this copolymer, the blocks A of a vinylaromatic compound constitute regions called domain and hence have a structure serving like crosslinking to thereby heighten the cohesive force of the polymer. In this structure, the domains are disposed so as to surround the blocks of a conjugated diene compound serving as a rubber ingredient. The larger the number of terminal polymer blocks A, the larger the number of domains by which the rubber ingredient serving as a wetting component is surrounded. Such a block copolymer is hence thought to be effective in preventing the penetration of the pressure-sensitive adhesive through fibers of an adherend and in thus enabling the pressure-sensitive adhesive to be peeled off lightly.

Examples of the above-described polymer include the block copolymers available under the trade names of Quintac SH-108 (manufactured by Nippon Zeon Co., Ltd.; styrene-isoprene-styrene block copolymer; number of terminal polymer blocks A, 3), Quintac 3450 (manufactured by Nippon Zeon Co., Ltd.; styrene-isoprene-styrene block copolymer; number of terminal polymer blocks A, 3), Kraton D-1320X (manufactured by Shell Kagaku K.K.; styrene-isoprene-styrene block copolymer; number of terminal polymer blocks A, 8), and RP-6404 (manufactured by Shell Kagaku K.K.; styrene-isoprene-styrene block copolymer; number of terminal polymer blocks A, 3-4). However, the elastomer should not be construed as being limited thereto.

That is, in the present invention, the composition of the pressure-sensitive adhesive may be suitably designed so that the adhesive force of the pressure-sensitive adhesive at the time of re-peeling (releasing force) is up to 4 times, preferably up to 3 times, the initial adhesive force thereof as measured in the initial stage after application. It is preferred that the elastomer be suitably selected so as to satisfy at least one of the requirements: a block copolymer in which the content of the polymer block A consisting mainly of units derived from a vinylaromatic compound is 18% by weight or higher; a block copolymer having a degree of coupling of 90% or higher; and a radial block copolymer having three or more terminal polymer blocks A.

The re-peelable pressure-sensitive adhesive of the present invention is preferably a pressure-sensitive adhesive composition at least comprising the block copolymer elastomer and a tackifier resin. The amount of the tackifier resin is desirably from 20 to 200 parts by weight, especially from 30 to 160 parts by weight, per 100 parts by weight of the elastomer. If the amount of the tackifier resin is smaller than 20 parts by weight, there is a possibility that the modulus of elasticity of the pressure-sensitive adhesive decreases with the lapse of time or increasing temperature to lower the adhesive force, or that the pressure-sensitive adhesive shows poor tackiness and insufficient adhesive force from the beginning. On the other hand, if it exceeds 200 parts by weight, there is a possibility that the pressure-sensitive adhesive shows poor tackiness and insufficient adhesive property in a low-temperature range.

The tackifier resin is not particularly limited as long as it is solid or liquid at ordinary temperature. However, from the standpoint of preventing the cohesive force and modulus of elasticity of the pressure-sensitive adhesive from decreasing to thereby inhibit the adhesive force of the pressure-sensitive adhesive from increasing, the softening point of the tackifier resin, when it is solid, is preferably about from 60 to 120°C, especially from 90 to 100°C. Examples of such tackifier resins include petroleum resins such as Marukarez Series manufactured by Maruzen Petrochemical Co.,

Ltd., Japan and Escorez Series manufactured by Tornex Co., aliphatic resins such as Arkon M Series manufactured by Arakawa Chemical Industry, Ltd., Japan, and terpene resins such as Clearon Series manufactured by Yasuhara Chemical Co., Ltd., Japan.

If desired, an antioxidant, a softener, a colorant, a filler (e.g., titanium white, zinc flower, calcium carbonate, talc, white mica, or a pigment), and the like may be incorporated into the pressure-sensitive adhesive composition described above.

A preferred embodiment of the re-peelable pressure-sensitive adhesive of the present invention is one which does not contain a softener such as a paraffin oil, because this pressure-sensitive adhesive is relatively rigid and less apt to penetrate into the surface of an adherend even after storage, so that it has an effect that the increase in the adhesive force thereof is further diminished.

Since the pressure-sensitive adhesive of the above-described kind is suitable for use as a hot-melt adhesive, it has another advantage over solvent-based pressure-sensitive adhesives. That is, the above pressure-sensitive adhesive attains an improved production efficiency and is free from environmental pollution. The thickness of the pressure-sensitive adhesive layer is not particularly limited, but the preferred range thereof is usually about from 10 to 150 µm. In the case of application to garments or the like, the thickness of the pressure-sensitive adhesive layer is usually about from 10 to 60 µm, preferably about from 30 to 40 µm.

The pattern of the pressure-sensitive adhesive layer formed on the surface of a base material or adherend is not particularly limited, and the adhesive layer may be deposited either over the whole surface or partly in the form of lines, fibers, dots, etc. It is also possible to form the pressure-sensitive adhesive layer by screen printing as in printing characters or drawings. Although methods for forming the pressure-sensitive adhesive layer are not particularly limited, the pressure-sensitive adhesive composition is preferably applied by hot-melt coating. It is also possible to produce a pressure-sensitive adhesive tape having at least one dry edge for easy nipping.

The re-peelable pressure-sensitive adhesive of the present invention may be used in the form of a pressure-sensitive adhesive layer alone without any base material therefor. However, it is preferred to provide the pressure-sensitive adhesive in the form of a pressure-sensitive adhesive tape A or sheet obtained by forming the specific re-peelable pressure-sensitive adhesive 2 of the present invention on at least one side of an appropriate base material 1 as shown in Fig. 1. In the case of a tape or sheet having the pressure-sensitive adhesive on both sides, one side may be used for fixing the tape or sheet to an adherend and the other may be used for bonding to another adherend. The pressure-sensitive adhesive tape or sheet may be further provided with a separator (not shown) treated with a releasing agent for protecting the pressure-sensitive adhesive 2.

The base material is not particularly limited. Preferred examples thereof include nonwoven fabrics and plastic films including single-layer films of polyesters or polyolefins (e.g., polyethylene, polypropylene, and blends thereof) and laminates of such films. In the case where the base material is to be applied to a garment, a heat-sealable layer is preferably formed on at least one side thereof so as to enable thermal bonding. Preferred examples of the material of this heat-sealable layer include materials not tacky at ordinary temperature, such as ethylene-vinyl acetate copolymers, polyethylenes (including ultralow-density polyethylene), and ethylene-acrylic copolymers (e.g., EMMA and EAA).

Another possible embodiment is a pressure-sensitive adhesive tape as shown in Fig. 2, which comprises a base material 1, the re-peelable pressure-sensitive adhesive 2 of the present invention and an ordinary pressure-sensitive adhesive 3 both deposited on one side of the base material 1, and a releasing agent 4 deposited on the other side. This structure produces an effect that when the ordinary pressure-sensitive adhesive 3 is used for fixing the pressure-sensitive adhesive tape to, e.g., a paper diaper or another adherend, the remaining re-peelable pressure-sensitive adhesive 2 can be used for manual fastening and unfastening.

Still another possible embodiment is a pressure-sensitive adhesive tape as shown in Fig. 3, which is obtained by alternately depositing the re-peelable pressure-sensitive adhesive 2 and an ordinary pressure-sensitive adhesive 3 on a base material 1 and folding the resulting tereko type tape. This structure produces an effect that the pressure-sensitive adhesive tape is usable as a small folding-type fastening tape. A further possible embodiment is a pressure-sensitive adhesive tape as shown in Fig. 4, which is obtained by depositing the re-peelable pressure-sensitive adhesive 2 and an ordinary pressure-sensitive adhesive 3 on one side of a base material 1 in one end part thereof and in the other end part thereof, respectively, folding the resulting structure into the shape of Z, and fixing part of the folded structure by heat sealing 5. This pressure-sensitive adhesive tape produces an effect that fixing is easy and this is usable for fixing like a string.

Still a further possible embodiment is a pressure-sensitive adhesive sheet for transfer as shown in Fig. 5, which is obtained by depositing the re-peelable pressure-sensitive adhesive 2 of the present invention on a separator 6 through a releasing agent 4, e.g., a silicone.

The present invention furthermore provides a fastening system for a disposable diaper, which system comprises using the above-described pressure-sensitive adhesive tape for fastening and re-fastening by applying the adhesive tape to the surface of the back sheet of the diaper. As shown in Fig. 6, in the case of the fastening system for a disposable diaper, the back sheet B having no so-called frontal tape (reinforcing film) serves as an adherend and

the re-peelable pressure-sensitive adhesive of the present invention functions as the pressure-sensitive adhesive of a fastener tape C.

The back sheet serving as an adherend in this fastening system of the disposable diaper is usually a film or sheet of a polyolefin plastic. Examples of the polyolefin include polyethylene and polypropylene. Also preferred are composites which are laminates of such a polyolefin film as the front-side layer with paper, a fabric, a nonwoven fabric, etc. A porous film or sheet having moisture permeability is also usable as the back sheet. If desired, a reinforcing tape may be applied to the back side of the back sheet B. This is effective in preventing back sheet breakage because only the part to which the fastener tape is applied can be reinforced.

The re-peelable pressure-sensitive adhesive and pressure-sensitive adhesive tape or sheet of the present invention are not particularly limited in applications thereof. Besides the above-described fastening system for disposable diapers, the pressure-sensitive adhesive and the pressure-sensitive adhesive tape or sheet are usable, for example, for sealing a sanitary napkin package (Fig. 7), for fixing a separator-less sanitary napkin (Fig. 8), for fixing a disposable pocket heater (Fig. 9), for opening and closing a package (Fig. 10), and for closing a plastic bag (Fig. 11). In these applications, the pressure-sensitive adhesive or the pressure-sensitive adhesive tape or sheet can be easily re-peeled. Moreover, the re-peelable pressure-sensitive adhesive of the present invention is also usable in producing a pressure-sensitive adhesive tape roll not coated with a releasing agent (Fig. 12). The tape roll thus obtained can be rewound lightly. In Fig. 7, A: a pressure-sensitive adhesive tape and D: a polyethylene sheet for napkin package. In Fig. 8, D: a polyethylene sheet for napkin package, E: a napkin, and 2: a re-peelable pressure-sensitive adhesive. In. Fig. 9, 1: a porous material, 2: a re-peelable pressure-sensitive adhesive, A: a pressure-sensitive adhesive sheet, and J: a heat generator. In Fig. 10, A: a pressure-sensitive adhesive tape and F: a package. In Fig. 11, A: a pressure-sensitive adhesive sheet and G: a plastic bag. In Fig. 12, H: a pressure-sensitive adhesive tape roll.

According to the present invention, it is possible to obtain a re-peelable pressure-sensitive adhesive, a pressure-sensitive adhesive tape or sheet, or a fastening system employing these with each of which satisfactory bonding and fixing with sufficient holding power and easy re-peeling after use or for position changing can be attained due to the use of a pressure-sensitive adhesive which after application undergoes less increase in adhesive force with the lapse of time or a temperature change, i.e., a pressure-sensitive adhesive which has less difference between the initial adhesive force just after application and the adhesive force at the time of re-peeling, removal after use, etc.

The present invention will be explained below by reference to Examples, but the invention should not be construed as being limited to these Examples. In the following Examples and Comparative Examples, all parts are given by weight.

EXAMPLES

In each of the Examples and Comparative Examples, the pressure-sensitive adhesive composition prepared according to the following formulation was dissolved into toluene, and the solution was applied on a 38 µm-thick polyester film in such an amount as to give a pressure-sensitive adhesive layer having a dry thickness of 30 µm and dried to obtain a pressure-sensitive adhesive sheet.

EXAMPLE 1

| | |
|---|---|
| Styrene-isoprene-styrene block copolymer (degree of coupling, 99% or higher; styrene content, 18 wt%; number of terminal styrene blocks, 2; Vector V4111D; manufactured by Tornex Co.) | 100 parts |
| Petroleum resin (softening point, 95°C; Marukarez H-700F; manufactured by Maruzen Petrochemical Co., Ltd.) | 50 parts |
| Antioxidant (Irganox; manufactured by Ciba Geigy Ltd.) | 2 parts |

EXAMPLE 2

| | |
|---|---|
| Styrene-isoprene-styrene block copolymer (degree of coupling, 90%; styrene content, 10 wt%; number of terminal styrene blocks, 3-4; Kraton D-1320X; manufactured by Shell Kagaku K.K.) | 100 parts |
| Aliphatic resin (softening point, 100°C; Arkon M100; manufactured by Arakawa Chemical Industry, Ltd.) | 40 parts |
| Antioxidant (Irganox; manufactured by Ciba Geigy Ltd.) | 2 parts |

EXAMPLE 3

| | |
|---|---|
| Styrene-isoprene-styrene block copolymer (degree of coupling, 99% or higher; styrene content, 30 wt%; number of terminal styrene blocks, 2; Vector V4211D; manufactured by Tornex Co.) | 100 parts |
| Petroleum resin (softening point, 95°C; Marukarez H-700F; manufactured by Maruzen Petrochemical Co., Ltd.) | 40 parts |
| Antioxidant (Irganox; manufactured by Ciba Geigy Ltd.) | 2 parts |

EXAMPLE 4

| | |
|---|---|
| Styrene-isoprene-styrene block copolymer (degree of coupling, 80%; styrene content, 30 wt%; number of terminal styrene blocks, 2; RP-6405; manufactured by Shell Kagaku K.K.) | 100 parts |
| Petroleum resin (softening point, 90°C; Arkon M90; manufactured by Arakawa Chemical Industry, Ltd.) | 50 parts |
| Antioxidant (Irganox; manufactured by Ciba Geigy Ltd.) | 2 parts |

EXAMPLE 5

| | |
|---|---|
| Styrene-isoprene-styrene block copolymer (degree of coupling, 70%; styrene content, 30 wt%; number of terminal styrene blocks, 3-4; RP-6404; manufactured by Shell Kagaku K.K.) | 100 parts |
| Petroleum resin (softening point, 95°C; Marukarez H-700F; manufactured by Maruzen Petrochemical Co., Ltd.) | 180 parts |
| Antioxidant (Irganox; manufactured by Ciba Geigy Ltd.) | 2 parts |

EXAMPLE 6

| | |
|---|---|
| Styrene-isoprene-styrene block copolymer (degree of coupling, 60%; styrene content, 25 wt%; number of terminal styrene blocks, 3; Quintac SH-108; manufactured by Nippon Zeon Co., Ltd.) | 100 parts |
| Petroleum resin (softening point, 95°C; Marukarez H-700F; manufactured by Maruzen Petrochemical Co., Ltd.) | 40 parts |
| Antioxidant (Irganox; manufactured by Ciba Geigy Ltd.) | 2 parts |

COMPARATIVE EXAMPLE 1

| | |
|---|---|
| Styrene-isoprene-styrene block copolymer (degree of coupling, 22%; styrene content, 15 wt%; number of terminal styrene blocks, 2; Quintac SL-113; manufactured by Nippon Zeon Co., Ltd.) | 100 parts |
| Petroleum resin (softening point, 95°C; Marukarez H-700F; manufactured by Maruzen Petrochemical Co., Ltd.) | 40 parts |
| Antioxidant (Irganox; manufactured by Ciba Geigy Ltd.) | 2 parts |

COMPARATIVE EXAMPLE 2

| | |
|---|---|
| Styrene-isoprene-styrene block copolymer (degree of coupling, 48%; styrene content, 17 wt%; number of terminal styrene blocks, 2; Quintac 3433; manufactured by Nippon Zeon Co., Ltd.) | 100 parts |

(continued)

| | |
|---|---|
| Petroleum resin (softening point, 95°C; Marukarez H-700F; manufactured by Maruzen Petrochemical Co., Ltd.) | 40 parts |
| Antioxidant (Irganox; manufactured by Ciba Geigy Ltd.) | 2 parts |

Evaluation

The pressure-sensitive adhesive tapes obtained in the Examples and Comparative Examples were examined for initial adhesive force and adhesive force after storage (with heating) by the following methods. The results obtained are shown in Table 1.

Initial Adhesive Force

Each pressure-sensitive adhesive tape was applied to a polyethylene film (thickness, 200 μm) by pressing the tape against the film by rolling a 2-kg roller (i.e., at 2 kg/cm) forward and backward once in a 23°C atmosphere. Thirty minutes after the application, the 180° peeling strength thereof (peel rate, 0.3 m/min) was measured.

Adhesive Force after Storage (with heating)

Each pressure-sensitive adhesive tape was applied to a polyethylene film (thickness, 200 μm) by pressing the tape against the film by rolling a 2-kg roller forward and backward once in a 23°C atmosphere. After the laminate sample was stored at 50°C for each of 3 days and 7 days, the 180° peeling strength thereof (peel rate, 0.3 m/min) was measured in a 23°C atmosphere.

Table 1

| Example No. | Initial Adhesive Force | Adhesive Force After Storage | | Increase in Adhesive Force | |
|---|---|---|---|---|---|
| | | 50°C x 3 days | 50°C x 7 days | 50°C x 3 days | 50°C x 7 days |
| | (g/25 mm) | (g/25 mm) | (g/25 mm) | (times) | (times) |
| Example 1 | 260 | 260 | 280 | 1.00 | 1.08 |
| Example 2 | 340 | 370 | 350 | 1.09 | 1.03 |
| Example 3 | 300 | 420 | 385 | 1.40 | 1.28 |
| Example 4 | 300 | 300 | 300 | 1.00 | 1.00 |
| Example 5 | 230 | 246 | 274 | 1.07 | 1.19 |
| Example 6 | 445 | 450 | 460 | 1.01 | 1.03 |
| Comparative Example 1 | 1200 | 5780 | 5000 | 4.82 | 4.17 |
| Comparative Example 2 | 910 | 3800 | 6200 | 4.18 | 6.81 |

Adhesive Force after Storage

Each pressure-sensitive adhesive tape was applied to a polyethylene film (thickness, 200 μm) by pressing the tape against the film by rolling a 2-kg roller forward and backward once in a 23°C atmosphere. After the laminate sample was stored at 23°C for each of 1 month, 6 months, and 30 months, the 180° peeling strength thereof (peel rate, 0.3 m/min) was measured. The results obtained are shown in Table 2.

Table 2

| Example No. | Initial Adhesive Force (g/25 mm) | Adhesive Force After Storage (g/25 mm) | | | Increase in Adhesive Force through 30 months (times) |
|---|---|---|---|---|---|
| | | after 1 month | after 6 months | after 30 months | |
| Example 1 | 260 | 270 | 270 | 260 | 1.00 |

Table 2 (continued)

| Example No. | Initial Adhesive Force (g/25 mm) | Adhesive Force After Storage (g/25 mm) | | | Increase in Adhesive Force through 30 months (times) |
|---|---|---|---|---|---|
| | | after 1 month | after 6 months | after 30 months | |
| Example 2 | 340 | 330 | 350 | 360 | 1.06 |
| Example 3 | 300 | 360 | 410 | 420 | 1.40 |
| Example 4 | 300 | 300 | 300 | 300 | 1.00 |
| Example 5 | 230 | 240 | 250 | 260 | 1.13 |
| Example 6 | 445 | 450 | 620 | 1080 | 2.42 |
| Comparative Example 1 | 1200 | 3600 | 6200 | 7200 | 6.00 |
| Comparative Example 2 | 910 | 4200 | 7500 | 8000* | 8.79* |

*) with film elongation

Measurement of Holding Power

Each pressure-sensitive adhesive tape was applied to a polyethylene film (50x80 $mm^2$; thickness, 100 µm) in such a manner as to result in an adhesion area of 25x25 $mm^2$. A load of 600 g was attached to the applied tape, and the time required for the loaded tape to fall off in a 40°C atmosphere was measured. As a result, the time was 200 minutes or longer for all the Examples and Comparative Examples.

Relationship between Peel Rate and Adhesive Force (Releasing Force)

The samples for initial adhesive force measurement which had been obtained in Example 1 and Comparative Example 1, i.e., the samples each obtained by applying the pressure-sensitive adhesive sheet to an adherend (200-µm polyethylene film) with application of pressure by rolling a 2-kg roller forward and backward once in a 23°C atmosphere, were allowed to stand at 60°C for 1 hour, and the 180° peeling strength of each sample was measured with a tensile tester at various peel rates and at 23°C and 63% RH. The results obtained are summarized in Fig. 13. In Fig. 13, lines a and b show the results of Example 1 and Comparative Example 1, respectively. These results show that in the case of the re-peelable pressure-sensitive adhesive sheet of the present invention (Example 1), the adhesive force to the adherend (releasing force) decreased with increasing peel rate when the peel rate was not lower than about 0.1 m/min. Hence, this re-peelable pressure-sensitive adhesive sheet can be easily peeled off in practical use.

On the other hand, in the case of the pressure-sensitive adhesive sheet of Comparative Example 1, the adhesive force to the adherend (releasing force) increased abruptly with increasing peel rate when the peel rate was not lower than about 0.1 m/min. Hence, this pressure-sensitive adhesive sheet is difficult to peel off in practical use.

Re-peelability

Each of the samples for initial adhesive force measurement which had been obtained in Examples 1 to 3 and Comparative Example 4 given later was applied to an adherend (200-µm polyethylene film) by pressing the sample against the adherend by rolling a 2-kg roller forward and backward once in a 23°C atmosphere. Within 3 minutes after the application, the 180° peeling strength of the sample was repeatedly measured with a tensile tester at a peel rate of 0.3 m/min and at 23°C and 60% RH.

The results obtained are shown in Table 3. The percentage decrease in adhesive force was determined using the following equation.

$$\text{Percentage decrease (\%)} = \left(1 - \frac{\text{Adhesive force at 3rd measurement}}{\text{Adhesive force at 1st measurement}}\right) \times 100$$

The resistance to separation, which is the ability of the pressure-sensitive adhesive tape to withstand separation when pulled in a shear direction, was evaluated based on the following criteria.

A: not separated
B: partly separated
C: separated

Table 3

| Example No. | Adhesive Force (g/25 mm) | | | Percentage Decrease (%) | Resistance to Separation |
|---|---|---|---|---|---|
| | 1st | 2nd | 3rd | | |
| Example 1 | 260 | 250 | 240 | 8 | A |
| Example 2 | 340 | 330 | 300 | 12 | A |
| Example 3 | 300 | 250 | 210 | 30 | A-B |
| Comparative Example 4 | 410 | 310 | 180 | 56 | C |

EXAMPLE 7

| | |
|---|---|
| Styrene-isoprene-styrene block copolymer (manufactured by Nippon Zeon Co., Ltd.; radial type; SH-108; degree of coupling, 60%; styrene content, 25%; number of terminal styrene blocks, 3) | 100 parts |
| Hydrogenated petroleum resin (manufactured by Maruzen Petrochemical Co., Ltd.; Marukarez) | 100 parts |
| Liquid terpene resin (manufactured by Yasuhara Chemical Co., Ltd.; YS Resin) | 10 parts |
| Antioxidant (manufactured by Ciba Geigy Ltd.; Irganox 1010) | 2 parts |

A base material sheet consisting of a polyethylene layer (80 µm thick) and formed on one side thereof a heat-sealable layer (20 µm thick) made of an ethylene-vinyl acetate copolymer (vinyl acetate content, 15%) was coated on the polyethylene layer side with the pressure-sensitive adhesive (hot-melt type) prepared according to the above formulation, at a thickness of 30 µm using a hot-melt coater. Thus, a re-peelable pressure-sensitive adhesive sheet of the present invention was obtained.

COMPARATIVE EXAMPLE 3

| | |
|---|---|
| Poly(2-ethylhexyl acrylate) | 100 parts |
| Isocyanate crosslinking agent | 4 parts |

A pressure-sensitive adhesive having the above composition was mixed with toluene, and the mixture was applied on the same base material sheet as in Example 7 at a thickness of 30 µm to obtain a pressure-sensitive adhesive sheet.

COMPARATIVE EXAMPLE 4

| | |
|---|---|
| Styrene-isoprene-styrene block copolymer (manufactured by Shell Kagaku K.K.; Kraton D-1107; degree of coupling, 85%; styrene content, 15%; number of terminal styrene blocks, 2) | 100 parts |
| Hydrogenated petroleum resin (manufactured by Maruzen Petrochemical Co., Ltd.; Marukarez) | 80 parts |
| Antioxidant (manufactured by Ciba Geigy Ltd.; Irganox 1010) | 2 parts |

A pressure-sensitive adhesive having the above composition was prepared in the same manner as in Example 7. This pressure-sensitive adhesive was applied on a polyester film (about 40 µm thick) as a base material sheet at a thickness of 30 µm, and the coating was heated at 50 to 80°C to remove the toluene to thereby obtain a pressure-sensitive adhesive sheet.

COMPARATIVE EXAMPLE 5

| | |
|---|---|
| Styrene-isoprene-styrene block copolymer (manufactured by Nippon Zeon Co., Ltd.; Quintac SL-113; degree of coupling, 20%; styrene content, 15%; number of terminal styrene blocks, 2) | 100 parts |
| Hydrogenated petroleum resin (manufactured by Maruzen Petrochemical Co., Ltd.; Marukarez) | 100 parts |
| Softener (paraffin oil) | 10 parts |
| Antioxidant (manufactured by Ciba Geigy Ltd.; Irganox 1010) | 2 parts |

The pressure-sensitive adhesive prepared according to the above formulation (hot-melt type) was applied with a hot-melt coater on the same base material as in Example 7 at a thickness of 30 μm to obtain a pressure-sensitive adhesive sheet.

Evaluation

The pressure-sensitive adhesive sheets obtained in the Example and Comparative Examples given above were evaluated by the following methods. The adherends used were: 1 ... Gunze YG Underwear; 2 ... standard cotton fabric (JIS); and 3 ... polyester (70)/cotton (30).

Initial Adhesive Force

Each pressure-sensitive adhesive sheet was applied to each adherend by pressing the pressure-sensitive adhesive sheet against the adherend by rolling a 2-kg roller forward and backward once in a 23°C atmosphere. After each sample was allowed to stand at 23°C for one day, the 180° peeling strength thereof was measured at 23°C with a tensile tester at a peel rate of 0.3 m/min. The results obtained are shown in Table 4.

Adhesive Force after Use (Releasing Force)

(a) Adhesive force was measured after 1-day storage at 60°C under a load of 5 kg per 25 mm in width × 200 mm in length of each sample in the same manner as the above.
(b) Adhesive force was measured after 6-month standing at ordinary temperature (23°C) in the same manner as the above.

In both tests, the samples were allowed to stand in a 23°C atmosphere for one day, before the adhesive force thereof was measured at 23°C. The results obtained are summarized in Table 4. The results show that the pressure-sensitive adhesive sheet of the present invention underwent less increase in adhesive force after use.

Table 4

| | Adherend | Example 7 | Comparative Example 3 | Comparative Example 4 | Comparative Example 5 |
|---|---|---|---|---|---|
| Initial Adhesive Force (g/25 mm) | 1 | 400 | 280 | 300 | 350 |
| | 2 | 380 | 180 | 210 | 290 |
| | 3 | 110 | 50 | 60 | 100 |
| Releasing Force after Use (g/25 mm) | | | | | |
| Condition (a) | 1 | 930 (2.3 times) | 1740 (6.2 times) | 1620 (5.4 times) | 1820 (5.2 times) |
| | 2 | 880 (2.3 times) | 1090 (6.0 times) | 1200 (5.7 times) | 1700 (5.9 times) |
| | 3 | 330 (3.0 times) | 500 (10 times) | 320 (5.3 times) | 880 (8.8 times) |
| Condition (b) | 1 | 510 (1.3 times) | 1790 (6.4 times) | 1510 (5.0 times) | 1900 (5.4 times) |
| | 2 | 430 (1.1 times) | 1200 (6.7 times) | 1310 (6.2 times) | 1620 (5.6 times) |
| | 3 | 400 (3.6 times) | 500 (10 times) | 420 (7.0 times) | 920 (9.2 times) |

Relationship between Peel Rate and Adhesive Force (Releasing Force)

The samples for initial adhesive force measurement, i.e., the samples each obtained by applying the pressure-sensitive adhesive sheet to an adherend (Gunze YG Underwear) with application of pressure by rolling a 2-kg roller forward and backward once in a 23°C atmosphere, were allowed to stand at 60°C for 1 hour, and the 180° peeling strength of each sample was measured at 23°C with a tensile tester at various peel rates. The results obtained are summarized in Table 5.

Table 5

| Peel Rate (m/min) | Adhesive Force (Releasing Force) (g/25 mm) | | |
|---|---|---|---|
| | Example 7 | Comparative Example 3 | Comparative Example 5 |
| 0.003 | 520 | 570 | 420 |
| 0.03 | 610 | 720 | 590 |
| 0.3 | 580 | 910 | 880 |
| 3 | 500 | 1020 | 1100 |
| 30 | 390 | 1150 | 1200 |
| 50 | 370 | 1230 | 1280 |

These results show that in the case of the pressure-sensitive adhesive sheet of the present invention, the adhesive force to the adherend (releasing force) decreased with increasing peel rate when the peel rate was not lower than 3 m/min. Hence, this pressure-sensitive adhesive sheet can be easily peeled off in practical use.

## Claims

1. A re-peelable pressure-sensitive adhesive which is applicable to an adherent and re-peelable therefrom, comprising an elastomer and a tackifier resin, wherein the elastomer is a block copolymer comprising a polymer block A consisting mainly of units derived from a vinylaromatic compound and a polymer block B consisting mainly of units derived from a conjugated diene compound,
**characterized in that**
said block copolymer has a block A content of 18% by weight or higher, or/and has a degree of coupling of 90% or higher, or/and is a radial block copolymer which has three or more terminal polymer blocks A.

2. The re-peelable pressure-sensitive adhesive according to claim 1, wherein the adhesive force (releasing force) of said pressuresensitive adhesive in peeling from the adherend is not higher than four times the initial adhesive force thereof as measured in the initial stage after application.

3. The re-peelable pressure-sensitive adhesive according to claims 1 or 2, wherein the adhesive force of the pressure-sensitive adhesive in peeling from the adherend at a peel rate of 3 m/min or higher decreases with increasing peel rate.

4. The re-peelable pressure-sensitive adhesive according to claims 1 or 2, wherein the adhesive force of the pressure-sensitive adhesive at a peel rate of 10 m/min or higher is equal to or lower than the adhesive force of the pressure-sensitive adhesive at a peel rate of 0.1 m/min or lower.

5. Use of the adhesive according to claims 1 to 4 for a fastening system for a disposable diaper having a polyolefin back sheet said adhesive being used for fastening the diaper by applying the re-peelable pressure-sensitive adhesive to the back sheet as an adherent.

6. Use of the adhesive according to claims 1 to 4 for a fastening system for a disposable pocket heater, said adhesive being used for fastening the pocket heater by applying the pressure-sensitive adhesive to an adherent.

7. The use of claim 6, wherein the adherent is a garment.

8. Use of the adhesive according to claims 1 to 4 for a fastening system for a sanitary napkin, the adhesive being used for sealing the napkin itself or a package thereof.

## Patentansprüche

1. Abziehbarer auf Druck ansprechender Kleber, der an ein Anklebeteil anbringbar und von diesem abziehbar ist, ein Elastomer und ein Klebrigmacher-Harz umfasst, wobei das Elastomer ein Blockcopolymer ist, das ein vorwiegend aus von einer vinylaromatischen Verbindung abgeleiteten Einheiten bestehendes Blockpolymer A und ein vorwiegend aus von einer konjugierten Dien-Verbindung abgeleiteten Einheiten bestehendes Blockpolymers B umfasst,
**dadurch gekennzeichnet, dass**
das Blockcopolymer einen Block-A-Gehalt von 18 Gew.-% oder mehr, oder/und einen Kopplungsgrad von 90% oder höher hat, oder/und ein Radial-Blockcopolymer ist, das drei oder mehr End-Polymerblöcke A aufweist.

2. Abziehbarer auf Druck ansprechender Kleber nach Anspruch 1, bei dem die Klebekraft (Lösekraft) des auf Druck ansprechenden Klebers beim Abziehen von dem Anklebeteil nicht höher als das 4fache der in der Anfangsstufe nach Anwendung gemessenen Anfangsklebekraft desselben ist.

3. Abziehbarer, auf Druck ansprechender Kleber nach den Ansprüchen 1 oder 2, bei dem die Klebekraft des auf Druck ansprechenden Klebers beim Abziehen von dem Anklebeteil mit einer Abziehrate von 3 m/min oder höher sich mit zunehmender Abziehrate verringert.

4. Abziehbarer auf Druck ansprechender Kleber nach den Ansprüchen 1 oder 2, bei dem die Klebekraft des auf Druck ansprechenden Klebers bei einer Abziehrate von 10 m/min oder höher gleich oder niedriger als die Klebekraft des auf Druck ansprechenden Klebers bei einer Abziehrate von 0,1 m/min oder darunter ist.

5. Verwendung des Klebers nach den Ansprüchen 1 bis 4 für ein Befestigungssystem für eine Wegwerf-Windel mit einer Polyolefin-Rückschicht, welcher Kleber für das Befestigen der Windel durch Anwenden des abziehbaren, auf Druck ansprechenden Klebers an der als ein Anklebeteil dienenden hinteren Schicht verwendet wird.

6. Verwendung des Klebers nach den Ansprüchen 1 bis 4 für ein Befestigungssystem für einen Wegwerf-Taschenheizer, wobei der Kleber zum Befestigen des Taschenheizers durch Anwenden des auf Druck ansprechenden Klebers an einem Anklebeteil verwendet wird.

7. Verwendung nach Anspruch 6, wobei das Anklebeteil ein Kleidungsstück ist.

8. Verwendung des Klebers nach den Ansprüchen 1 bis 4 für ein Befestigungssystem für eine Damenbinde, wobei der Kleber zum Abdichten der Damenbinde selbst oder einer Packung derselben verwendet wird.

## Revendications

1. Adhésif sensible à la pression redécollable qui est applicable à un substrat d'adhérence et qui peut en être redécollé, comprenant un élastomère et une résine conférant l'adhésivité, où l'élastomère est un copolymère séquencé comprenant une séquence polymère A consistant principalement en unités dérivées d'un composé vinylaromatique et une séquence polymère B consistant principalement en unités dérivées d'un composé diène conjugué, caractérisé en ce que ledit copolymère séquencé a une teneur en séquences A de 18 % en masse ou plus et/ou a un degré de couplage de 90 % ou plus et/ou est un copolymère séquencé radial qui a trois ou plus de trois séquences polymères A terminales.

2. Adhésif sensible à la pression redécollable selon la revendication 1 dans lequel la force adhésive (force de séparation) dudit adhésif sensible à la pression lors du décollement d'avec le substrat d'adhérence n'est pas supérieure à quatre fois sa force adhésive initiale mesurée au stade initial après l'application.

3. Adhésif sensible à la pression redécollable selon les revendications 1 ou 2 dans lequel la force adhésive de l'adhésif sensible à la pression lors du décollement d'avec le substrat d'adhérence à une vitesse de décollement de 3 m/min ou plus décroît quand la vitesse de décollement croît.

4. Adhésif sensible à la pression redécollable selon les revendications 1 ou 2 dans lequel la force adhésive de l'adhésif sensible à la pression à une vitesse de décollement de 10 m/min ou plus est égale ou inférieure à la force adhésive de l'adhésif sensible à la pression à une vitesse de décollement de 0,1 m/min ou moins.

**5.** Utilisation de l'adhésif selon les revendications 1 à 4 pour un système de fixation pour une couche jetable ayant une feuille dorsale de polyoléfine, ledit adhésif étant utilisé pour fixer la couche par application de l'adhésif sensible à la pression redécollable à la feuille dorsale en tant que substrat d'adhérence.

**6.** Utilisation de l'adhésif selon les revendications 1 à 4 pour un système de fixation pour un dispositif chauffant de poche jetable, ledit adhésif étant utilisé pour fixer le dispositif chauffant de poche par application de l'adhésif sensible à la pression à un substrat d'adhérence.

**7.** Utilisation selon la revendication 6, où le substrat d'adhérence est un vêtement.

**8.** Utilisation de l'adhésif selon les revendications 1 à 4 pour un système de fixation pour une serviette hygiénique, l'adhésif étant utilisé pour fermer hermétiquement la serviette hygiénique elle-même ou un emballage de celle-ci.

Fig. 1

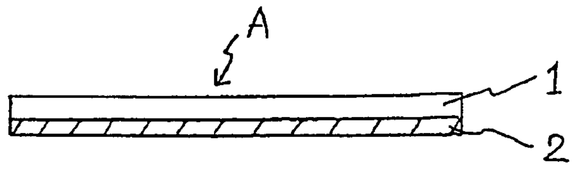


Fig. 2

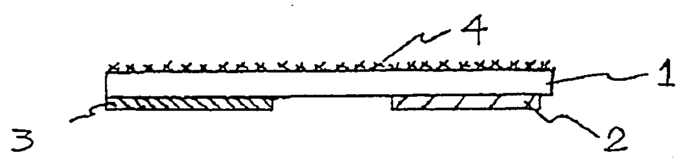


Fig. 3

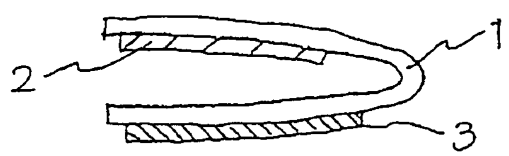


Fig. 4

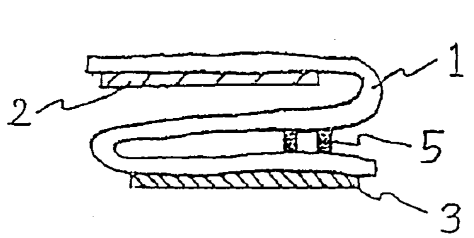

Fig. 5

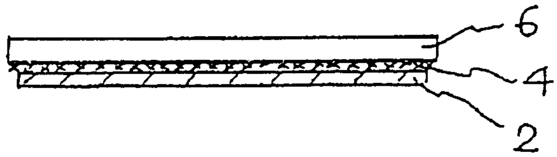


Fig. 6

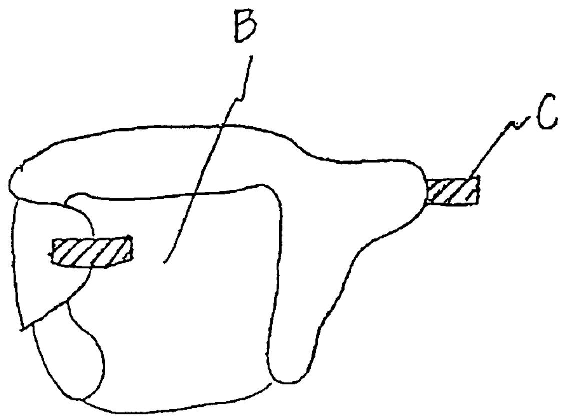


Fig. 7

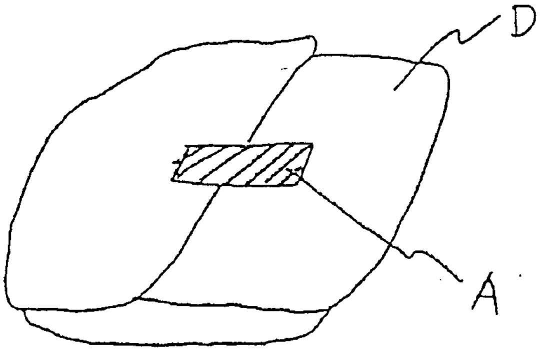

Fig. 8

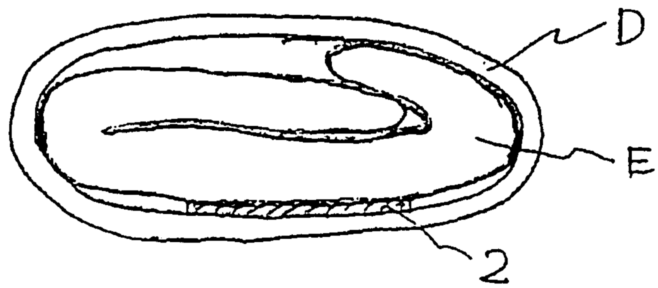


Fig. 9

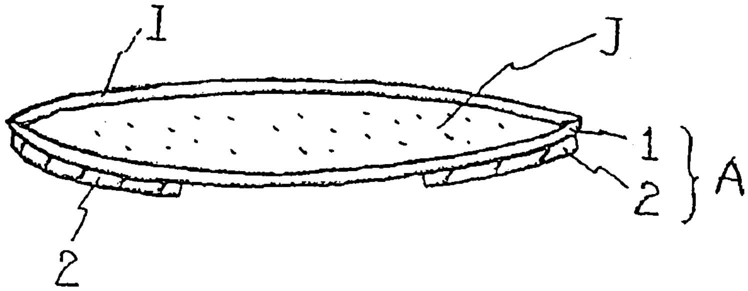


Fig. 10

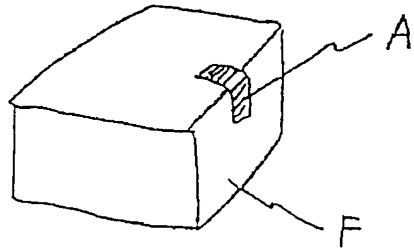

Fig. 11

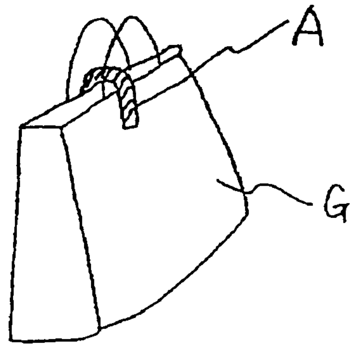


Fig. 12

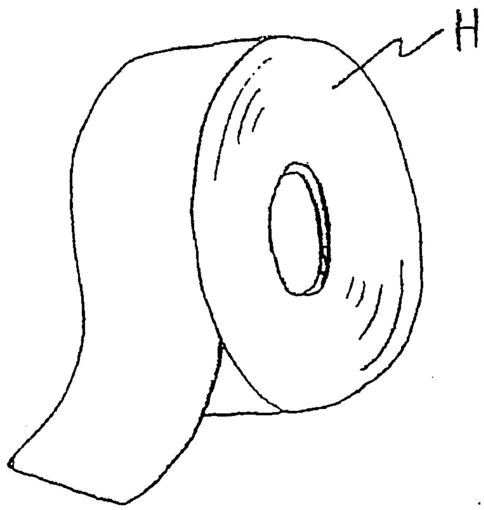

Fig. 13

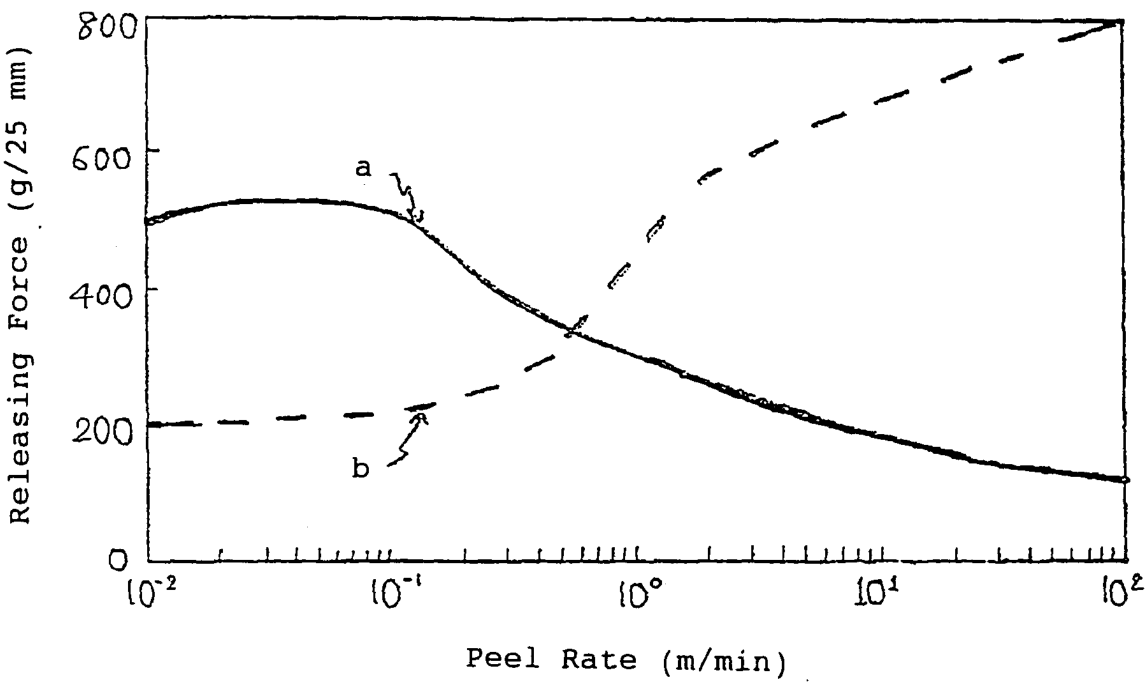
800
600
400
200
0
Releasing Force (g/25 mm)
a
b
$10^{-2}$
$10^{-1}$
$10^{0}$
$10^{1}$
$10^{2}$
Peel Rate (m/min)